# EUROPEAN PATENT SPECIFICATION

(11) **EP 4 144 286 B1**
(45) Date of publication and mention of the grant of the patent: **30.10.2024**
(21) Application number: 21194591.0
(22) Date of filing: 02.09.2021
(51) Int. Cl.: A61B 1/303, A61B 1/00

(54) **VAGINAL SPECULUM**
VAGINALSPEKULUM
SPÉCULUM VAGINAL

(43) Date of publication of application: 08.03.2023
(73) Proprietor: Tsai, Yih-Chiou, Taichung (TW)
(72) Inventor: Tsai, Yih-Chiou, Taichung (TW)
(74) Representative: Melchior, Robin

(56) References cited:
- GB-A- 2 578 723
- US-A1- 2020 069 171

## Description

### BACKGROUND

### 1. Field of the Invention

The present invention relates to a disposable medical device, and more particularly to a vaginal speculum that has a threaded rod free from rotating relative to a handle thereof.

### 2. Description of Related Art

A conventional vaginal speculum substantially has an upper member, a lower member, a threaded rod, and a nut. The upper member and the lower member are pivotally connected with each other. Each of the upper and the lower members has a handle extending downwardly therefrom. The threaded rod is fixed in the handle of the lower member and extends toward and through the handle of the upper member. The nut is connected with the threaded rod via a threaded connection and selectively abuts against the handle of the upper member to limit a relative opening position of the upper and the lower members.

To prevent the threaded rod from rotating relative to the lower member during rotating the nut to adjust a position of the nut relative to the threaded rod, the threaded rod has a limiting surface formed in an annular surface of a head thereof, and the lower member has an insertion cavity corresponding to the head of the threaded rod in shape and in size and formed in the handle thereof. The head of the threaded rod is mounted in and engages with the insertion cavity to prevent the threaded rod from rotating relative to the lower member. In addition, another conventional vaginal speculum is also disclosed in US 2020/069171 A1.

However, because the threaded rod and the lower member are made of plastic, the head of the threaded rod and the insertion cavity of the lower member are easily deformed via insertion molding. Consequently, the head of the threaded rod cannot be stably engaged with the insertion cavity of the lower member, the head of the threaded rod is easily disengaged from or separated from the insertion cavity, and the threaded rod is easily rotated relative to the lower member.

### SUMMARY OF THE INVENTION

To overcome the shortcomings, the present invention provides a vaginal speculum to mitigate or obviate the aforementioned problems.

The main objective of the present invention is to provide a vaginal speculum having a rod firmly engaged in the handle of the lower member.

A vaginal speculum comprises an upper member, a lower member, a threaded rod, and a nut. The upper member has a first handle extending downwardly from the upper member. The lower member is pivotally connected with the upper member and has a second handle extending downwardly from the lower member, located at a side of the first handle, and having a first side surface away from the first handle and a second side surface near the first handle. A positioning cavity is formed in the second handle and has an insertion cavity and multiple engaging grooves. The insertion cavity is recessed in the first side surface of the second handle and has an end surface away from the first side surface of the second handle spaced from the second side surface of the second handle and at least one cavity limiting surface defined in an annular surface of the insertion cavity.

The engaging grooves are arranged around the insertion cavity and are spaced from each other. Each of the engaging grooves has a side opening formed in the annular surface of the insertion cavity and communicating with the insertion cavity and an end surface near the first side surface of the second handle spaced from the first side surface of the second handle. The threaded rod has a head, multiple engaging protrusions, and a rod. The head is mounted in the insertion cavity of the positioning cavity and has at least one head limiting surface defined in an annular surface of the head and corresponding to the at least one cavity limiting surface in position and shape. The engaging protrusions protrude from the annular surface of the head, are spaced from each other, and are respectively engaged with the engaging grooves of the positioning cavity. The rod extends from the head toward the first handle and extends through the second handle and the first handle. The nut is connected with the rod of the threaded rod via a threaded connection and selectively abuts against the first handle.

Other objects, advantages, and novel features of the invention will become more apparent from the following detailed description when taken in conjunction with the accompanying drawings.

### BRIEF DESCRIPTION OF THE DRAWINGS

Fig. 1 is a perspective view of a vaginal speculum in accordance with the present invention;
Fig. 2 is an exploded perspective view of the vaginal speculum in Fig. 1 before assembly;
Fig. 3 is an enlarged perspective view of a threaded rod of the vaginal speculum in Fig. 1;
Fig. 4 is an enlarged side view of the threaded rod of the vaginal speculum in Fig. 3;
Fig. 5 is an enlarged end view of the threaded rod of the vaginal speculum in Fig. 3;
Fig. 6 is an enlarged perspective view of a lower member of the vaginal speculum in Fig. 1;
Fig. 7 is an enlarged perspective view in partial section of the lower member of the vaginal speculum in Fig. 6;
Fig. 8 is another enlarged perspective view of the lower member of the vaginal speculum in Fig. 6; and
Fig. 9 is an enlarged side view in partial section of the vaginal speculum in Fig. 1.

### DETAILED DESCRIPTION OF THE INVENTION

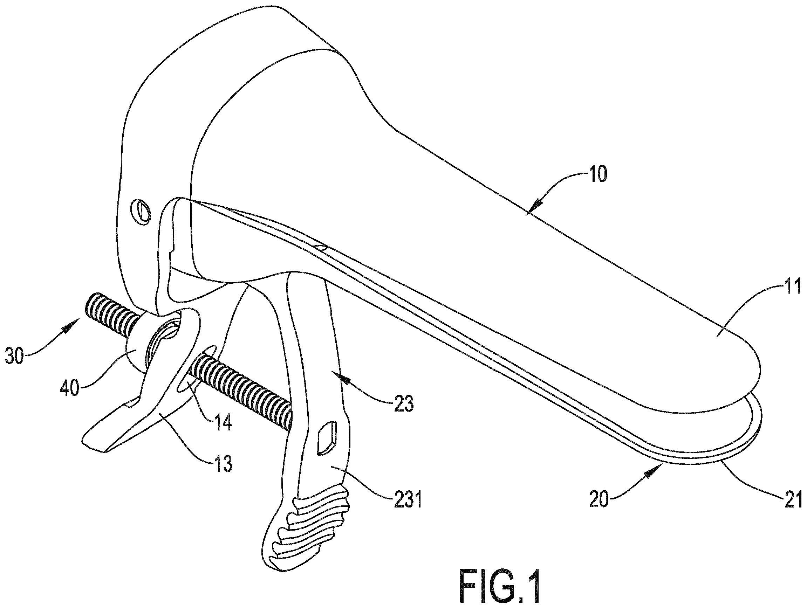

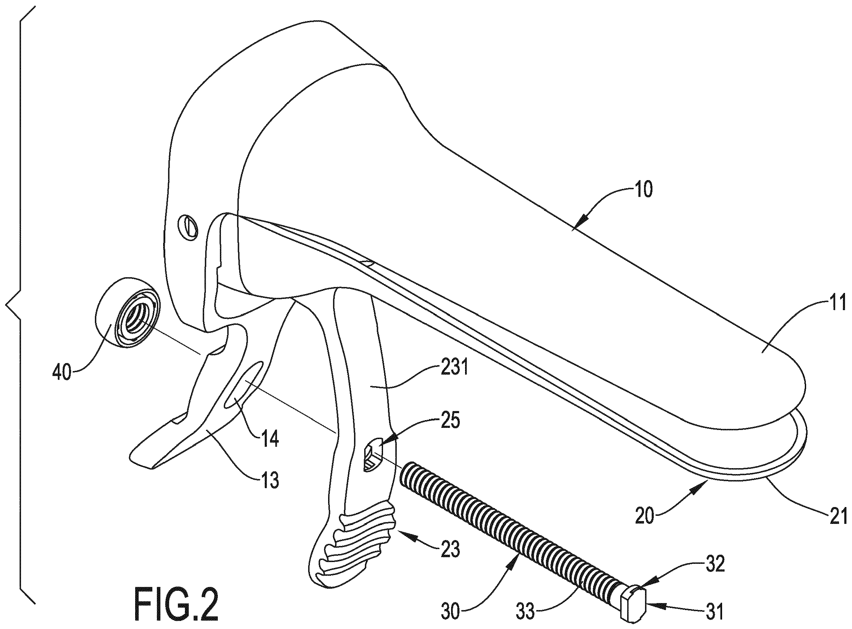

With reference to Figs. 1 and 2, a vaginal speculum in accordance with the present invention has an upper member 10, a lower member 20, a threaded rod 30, and a nut 40. The upper member 10, the lower member 20, the threaded rod 30, and the nut 40 are made of plastic.

The upper member 10 and the lower member 20 are pivotally connected with each other. The upper member 10 has an upper blade 11 formed at a front side of the upper member 10, the lower member 20 has a lower blade 21 formed at a front side of the lower member 20, and the upper and the lower blades 11, 21 can be moved away from or toward each other. The upper member 10 has a first handle 13 extending downwardly from the upper member 10. The lower member 20 has a second handle 23 extending from the lower member 20 and located at a side of the first handle 13. In this embodiment, the second handle 23 is located in front of the first handle 13. The first handle 13 and the second handle 23 are held and pressed by a user to move relative to each other to drive the upper blade 11 and the lower blade 21 to open relative to each other.

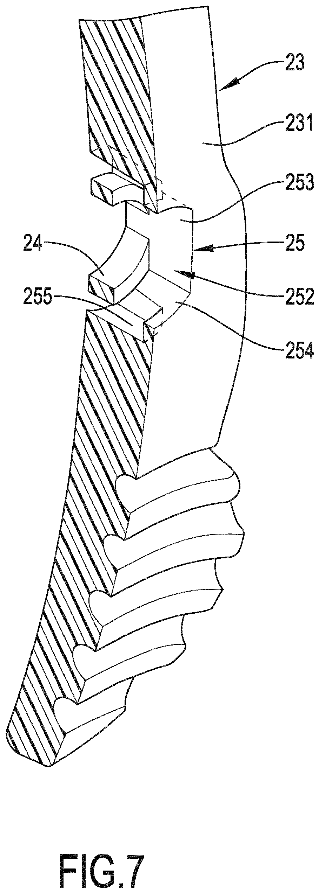

With reference to Figs. 2, 6, and 7, the second handle 23 has a first side surface 231 and a second side surface 232. The first side surface 231 is located away from the first handle 13. The second side surface 232 is located near the first handle 13. A positioning cavity 25 is formed in the second handle 23. The positioning cavity 25 has an insertion cavity 252 and multiple engaging grooves 255. The insertion cavity 252 is recessed in the first side surface 231 of the second handle 23. An end surface of the insertion cavity 252 away from the first side surface 231 is spaced from the second side surface 232. The insertion cavity 252 has at least one cavity limiting surface 253 defined in an annular surface of the insertion cavity 252. The engaging grooves 255 are arranged around the insertion cavity 252 and are spaced from each other. Each of the engaging grooves 255 has a side opening formed in the annular surface of the insertion cavity 252 and communicating with the insertion cavity 252. An end surface of each of the engaging grooves 255 near the first side surface 231 of the second handle 23 is spaced from the first side surface 231 of the second handle 23. Preferably, each of the engaging grooves 255 has an end opening formed in the second side surface 232 of the second handle 23.

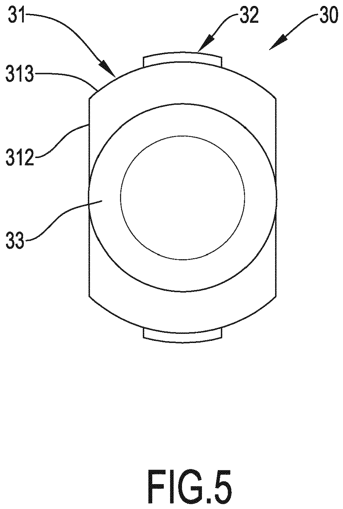

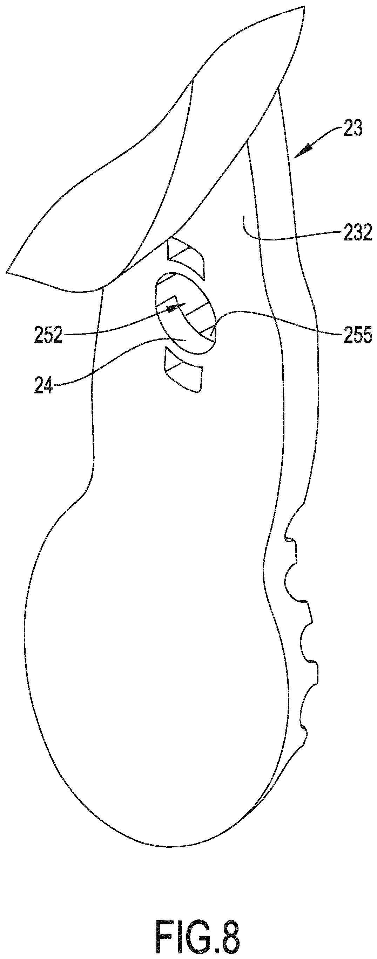

With reference to Figs. 2 to 5, and 8, the threaded rod 30 has a head 31, a rod 33, and multiple engaging protrusions 32. The head 31 is mounted in the insertion cavity 252 of the positioning cavity 25 and has at least one head limiting surface 312 defined in an annular surface of the head 31, corresponding to the at least one cavity limiting surface 253 in position and shape, and aligned with the at least one cavity limiting surface 253. The engaging protrusions 32 protrude from the annular surface of the head 31, are spaced from each other, and are respectively engaged with the engaging grooves 255 of the positioning cavity 25. The rod 33 extends from the head 31 toward the first handle 13 and extends through the second handle 23 and the first handle 13. A rod hole 24 is formed through the second side surface 232 of the second handle 23 and the end surface of the insertion cavity 252 and communicates with the insertion cavity 252. A diameter of a circumscribed circle of the rod hole 24 is smaller than a diameter of a circumscribed circle of the insertion cavity 252. The engaging grooves 255 are spaced from the rod hole 24. A through hole 14 is formed through the first handle 13. The rod 33 of the threaded rod 30 extends through the rod hole 24 in the second handle 23 and the through hole 14 in the first handle 13 and extends out from the first handle 13.

With reference to Figs. 1 and 2, the nut 40 is connected with the rod 33 of the threaded rod 30 via a threaded connection and selectively abuts against the first handle 13 to limit a relative open position of the first upper member 10 and the lower member 20.

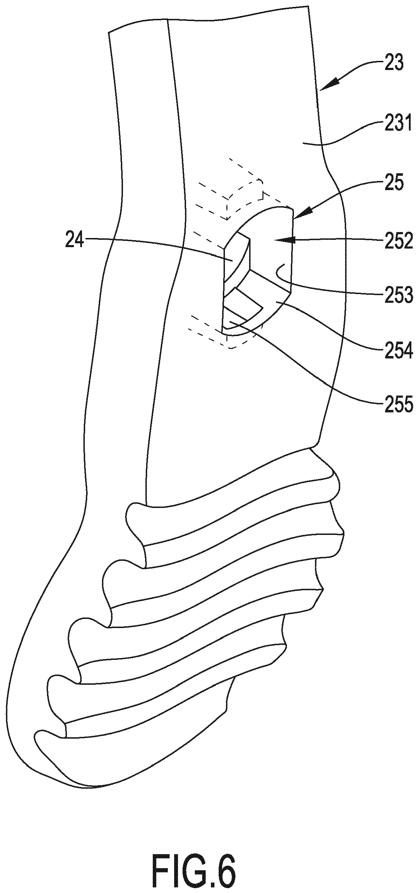

In this embodiment, with reference to Figs. 6 to 9, the insertion cavity 252 has two said cavity limiting surfaces 253 and two cavity arced surfaces 254 defined in the annular surface of the insertion cavity 252. The two cavity limiting surfaces 253 are spaced from each other. The two cavity arced surfaces 254 are connected with the two cavity limiting surfaces 253 and are arranged opposite to each other. The positioning cavity 25 has two said engaging grooves 255 being adjacent to the two cavity arced surfaces 254 respectively and communicating with the insertion cavity 252.

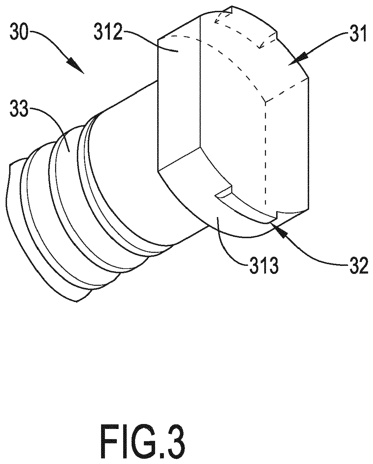

With reference to Figs. 3 to 5, and 9, the head 31 of the threaded rod 30 has two said head limiting surfaces 312 and two head arced surfaces 313 defined in the annular surface of the head 31. The two head limiting surfaces 312 are respectively aligned with the two cavity limiting surfaces 253. The two head arced surfaces 313 are connected with the two head limiting surfaces 312, are arranged opposite to each other, and are respectively aligned with the two cavity arced surfaces 254. The threaded rod 30 has two said engaging protrusions 32 respectively protruding from the two head arced surfaces 313 and respectively engaged with the two engaging grooves 255.

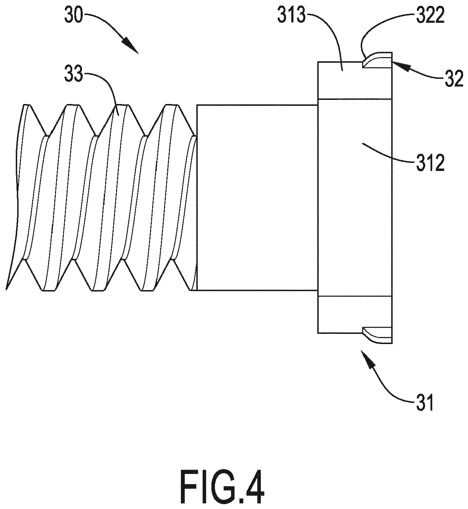

Preferably, as shown in Fig. 4, one of two ends of each of the engaging protrusions 32 away from the rod 33 is aligned with one of two ends of the head 31 away from the rod 33. A distance is formed between the other one of the two ends of each of the engaging protrusions 32 near the rod 33 and the other one of the two ends of the head 31 near the rod 33. A length of each of the engaging protrusions 32 is smaller than a length of the head 31. The length of each of the engaging protrusions 32 is smaller than or equal to 2/3 times of the length of the head 31 and is larger than or equal to 1/3 times of the length of the head 31.

Preferably, each of the engaging protrusions 32 has a guiding surface 322 defined in the end of the engaging protrusion 32 near the rod 33. The guiding surface 322 extends toward the rod 33 and gradually approaches the annular surface of the head 31. So the engaging protrusions 32 can be easily pressed into the insertion cavity 252.

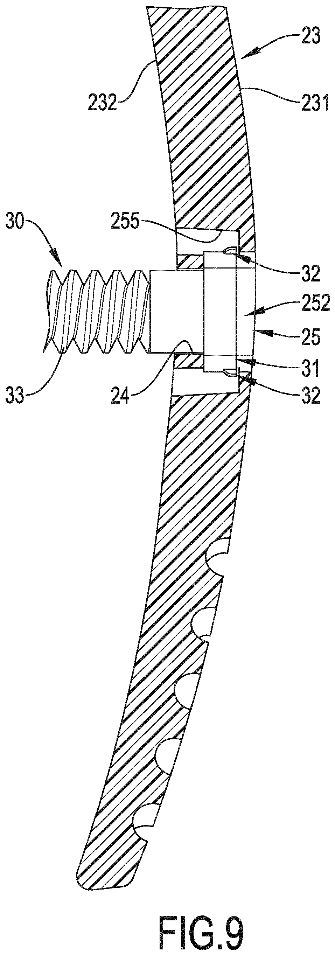

With reference to Figs. 2 and 9, to assemble the threaded rod 30, the rod 33 of the threaded rod 30 is inserted in the rod hole 24 in the second handle 23 from the first side surface 231, and the head 31 of the threaded rod 30 is pressed from the end thereof away from the rod 33 to press the engaging protrusions 32 of the threaded rod 30 into the insertion cavity 252 and slide to engage with the engaging grooves 255 respectively. The end of the head 31 of the threaded rod 30 near the rod 33 is limited by the end surface of the insertion cavity 252, and the ends of the engaging protrusions 32 away from the rod 33 are limited by the end surfaces of the engaging grooves 255 near the first side surface 231 of the second handle 23. So a longitudinal position of the threaded rod 30 relative to the second handle 23 is limited, and the threaded rod 30 will not be separated from the positioning cavity 25. Moreover, because the head 31 of the threaded rod 30 has the head limiting surfaces 312 corresponding to the cavity limiting surfaces 253 of the insertion cavity 252 in shapes and positions, the threaded rod 30 is prevented from rotating relative to the second handle 23. With such arrangement, the threaded rod 30 can be easily mounted in the second handle 23, and the head 31 of the threaded rod 30 can be firmly fixed in the positioning cavity 25 in the second handle 23.

## Claims

1. A vaginal speculum comprising:
an upper member (10) having a first handle (13) extending downwardly from the upper member (10);
a lower member (20) pivotally connected with the upper member (10) and having
a second handle (23) extending downwardly from the lower member (20), located at a side of the first handle (13) and having a first side surface (231) away from the first handle (13) and a second side surface (232) near the first handle (13); and
a positioning cavity (25) formed in the second handle (23) and having
an insertion cavity (252) recessed in the first side surface (231) of the second handle (23) and having an end surface away from the first side surface (231) of the second handle (23) spaced from the second side surface (232) of the second handle (23) and at least one cavity limiting surface (253) defined in an annular surface of the insertion cavity (252);
a threaded rod (30) having
a head (31) mounted in the insertion cavity (252) of the positioning cavity (25) and having
at least one head limiting surface (312) defined in an annular surface of the head (31) and corresponding to the at least one cavity limiting surface (253) in position and shape; and
a rod (33) extending from the head (31) toward the first handle (13) and extending through the second handle (23) and the first handle (13); and
a nut (40) connected with the rod (33) of the threaded rod (30) via a threaded connection and selectively abutting against the first handle (13); and **characterized in that**
the positioning cavity (25) has multiple engaging grooves (255) arranged around the insertion cavity (252) and spaced from each other, and each of the engaging grooves (255) having a side opening formed in the annular surface of the insertion cavity (252) and communicating with the insertion cavity (252) and an end surface near the first side surface (231) of the second handle (23) spaced from the first side surface (231) of the second handle (23); and
the threaded rod (30) has multiple engaging protrusions (32) protruding from the annular surface of the head (31), spaced from each other, and respectively engaged with the engaging grooves (255) of the positioning cavity (25) .

2. The vaginal speculum as claimed in claim 1, wherein each of the engaging grooves (255) has an end opening formed in the second side surface (232) of the second handle (23).

3. The vaginal speculum as claimed in claim 2, wherein each of the engaging protrusions (32) has a guiding surface (322) defined in one of two ends of the engaging protrusion (32) near the rod (33).

4. The vaginal speculum as claimed in claim 3, wherein one of the two ends of each of the engaging protrusions (32) away from the rod (33) is aligned with one of two ends of the head (31) away from the rod (33), and a distance is formed between the other one of the two ends of each of the engaging protrusions (32) near the rod (33) and the other one of the two ends of the head (31) near the rod (33) .

5. The vaginal speculum as claimed in any one of claims 1 to 4, wherein
the insertion cavity (252) has two said cavity limiting surfaces (253) and two cavity arced surfaces (254) defined in the annular surface of the insertion cavity (252), the two cavity limiting surfaces (253) are spaced from each other, and the two cavity arced surfaces (254) are connected with the two cavity limiting surfaces (253) and are arranged opposite to each other;
the positioning cavity (25) has two said engaging grooves (255) being adjacent to the two cavity arced surfaces (254) respectively and communicating with the insertion cavity (252) ;
the head (31) of the threaded rod (30) has two said head limiting surfaces (312) and two head arced surfaces (313), the two head limiting surfaces (312) are respectively aligned with the two cavity limiting surfaces (253), and the two head arced surfaces (313) are connected with the two head limiting surfaces (312), are arranged opposite to each other, and are respectively aligned with the two cavity arced surfaces (254); and
the threaded rod (30) has two said engaging protrusions (32) respectively protruding from the two head arced surfaces (313) and respectively engaged with the two engaging grooves (255).

6. The vaginal speculum as claimed in claim 5, wherein
a rod hole (24) is formed through the second side surface (232) of the second handle (23) and the end surface of the insertion cavity (252) and communicates with the insertion cavity (252);
a diameter of a circumscribed circle of the rod hole (24) is smaller than a diameter of a circumscribed circle of the insertion cavity (252);
the engaging grooves (255) are spaced from the rod hole (24);
a through hole (14) is formed through the first handle (13); and
the rod (33) of the threaded rod (30) extends through the rod hole (24) in the second handle (23) and the through hole (14) in the first handle (13) and extends out from the first handle (13).

## Patentansprüche

1. Vaginalspekulum, umfassend:
ein oberes Element (10) mit einem ersten Griff (13), der sich von dem oberen Element (10) nach unten erstreckt;
ein unteres Element (20), das drehbar mit dem oberen Element (10) verbunden ist und einen zweiten Griff (23) aufweist, der sich von dem unteren Element (20) nach unten erstreckt, sich auf einer Seite des ersten Griffs (13) befindet und eine erste Seitenfläche (231) entfernt vom ersten Griff (13) und eine zweite Seitenfläche (232) nahe dem ersten Griff (13) aufweist; und
einen Positionierungshohlraum (25), der in dem zweiten Griff (23) ausgebildet ist und einen Einführhohlraum (252) aufweist, der in erste Seitenfläche (231) des zweiten Griffs (23) eingesenkt ist und eine Endfläche entfernt von der ersten Seitenfläche (231) des zweiten Griffs (23) beabstandet von der zweiten Seitenfläche (232) des zweiten Griffs (23) und mindestens eine Hohlraumbegrenzungsfläche (253) aufweist, die in einer ringförmigen Fläche des Einführhohlraums (252) definiert ist;
eine Gewindestange (30) mit einem Kopf (31), der in den Einführhohlraum (252) des Positionierungshohlraums (25) montiert ist und mindestens eine Kopfbegrenzungsfläche (312) aufweist, die in einer ringförmigen Fläche des Kopfs (31) definiert ist und der mindestens einen Hohlraumbegrenzungsfläche (253) im Hinblick auf Lage und Form entspricht; und
eine Stange (33), die sich vom Kopf (31) in Richtung des ersten Griffs (13) erstreckt und durch den zweiten Griff (23) und den ersten Griff (13) verläuft; und
eine Mutter (40), die mit der Stange (33) der Gewindestange (30) über eine Gewindeverbindung verbunden ist und selektiv am ersten Griff (13) anliegt; und **dadurch gekennzeichnet ist, dass**
der Positionierungshohlraum (25) mehrere Eingriffsnuten (255) aufweist, die um den Einführhohlraum (252) angeordnet und voneinander beabstandet sind, und jede der Eingriffsnuten (255) eine seitliche Öffnung aufweist, die in der ringförmigen Fläche des Einführhohlraums (252) ausgebildet ist und mit dem Einführhohlraum (252) und einer Endfläche nahe der ersten Seitenfläche (231) des zweiten Griffs (23) beabstandet von der ersten Seitenfläche (231) des zweiten Griffs (23) in Verbindung steht; und
die Gewindestange (30) mehrere Eingriffsvorsprünge (32) aufweist, die von der ringförmigen Fläche des Kopfs (31) vorstehen, voneinander beabstandet sind und jeweils mit den Eingriffsnuten (255) des Positionierungshohlraums (25) im Eingriff sind.

2. Vaginalspekulum nach Anspruch 1, wobei jede der Eingriffsnuten (255) eine Endöffnung aufweist, die in der zweiten Seitenfläche (232) des zweiten Griffs (23) ausgebildet ist.

3. Vaginalspekulum nach Anspruch 2, wobei jeder der Eingriffsvorsprünge (32) eine Führungsfläche (322) aufweist, die in einem von zwei Enden des Eingriffsvorsprungs (32) nahe der Stange (33) ausgebildet ist.

4. Vaginalspekulum nach Anspruch 3,
wobei eines der zwei Enden von jedem der Eingriffsvorsprünge (32) entfernt von der Stange (33) mit einem von zwei Enden des Kopfs (31) entfernt von der Stange (33) ausgerichtet ist und ein Abstand zwischen dem anderen der zwei Enden von jedem der Eingriffsvorsprünge (32) nahe der Stange (33) und dem anderen der zwei Enden des Kopfs (31) nahe der Stange (33) gebildet ist.

5. Vaginalspekulum nach einem der Ansprüche 1 bis 4, wobei
der Einführhohlraum (252) zwei Hohlraumbegrenzungsflächen (253) und zwei Hohlraumwölbungsflächen (254) aufweist, die in der ringförmigen Fläche des Einführhohlraums (252) definiert sind, wobei die zwei Hohlraumbegrenzungsflächen (253) voneinander beabstandet sind und die zwei Hohlraumwölbungsflächen (254) mit den zwei Hohlraumbegrenzungsflächen (253) verbunden und einander gegenüberliegend angeordnet sind;
der Positionierungshohlraum (25) zwei Eingriffsnuten (255) aufweist, die jeweils an die zwei Hohlraumwölbungsflächen (254) angrenzen und mit dem Einführhohlraum (252) in Verbindung stehen;
der Kopf (31) der Gewindestange (30) zwei Kopfbegrenzungsflächen (312) und zwei Kopfwölbungsflächen (313) aufweist, wobei die zwei Kopfbegrenzungsflächen (312) jeweils mit den zwei Hohlraumbegrenzungsflächen (253) ausgerichtet sind und die zwei Kopfwölbungsflächen (313) mit den zwei Kopfbegrenzungsflächen (312) verbunden, einander gegenüberliegend angeordnet und jeweils mit den zwei Hohlraumwölbungsflächen (254) ausgerichtet sind; und
die Gewindestange (30) zwei Eingriffsvorsprünge (32) aufweist, die jeweils von den zwei Kopfwölbungsflächen (313) vorstehen und jeweils mit den zwei Eingriffsnuten (255) im Eingriff sind.

6. Vaginalspekulum nach Anspruch 5, wobei
ein Stangenloch (24) durch die zweite Seitenfläche (232) des zweiten Griffs (23) und die Endfläche des Einführhohlraums (252) ausgebildet ist und mit dem Einführhohlraum (252) in Verbindung steht;
ein Durchmesser eines Umkreises des Stangenlochs (24) kleiner als ein Durchmesser eines Umkreises des Einführhohlraums (252) ist;
die Eingriffsnuten (255) von dem Stangenloch (24) beabstandet sind;
ein Durchgangsloch (14) durch den ersten Griff (13) ausgebildet ist; und
die Stange (33) der Gewindestange (30) durch das Stangenloch (24) im zweiten Griff (23) und das Durchgangsloch (14) im ersten Griff (13) verläuft und sich vom ersten Griff (13) weg erstreckt.

## Revendications

1. Spéculum vaginal comprenant:
un élément supérieur (10) comportant une première poignée (13) s'étendant vers le bas à partir de l'élément supérieur (10);
un élément inférieur (20) connecté de façon pivotante à l'élément supérieur (10) et comportant
une deuxième poignée (23) s'étendant vers le bas à partir de l'élément inférieur (20), située à un côté de la première poignée (13) et comportant une première surface latérale (231) éloignée de la première poignée (13) et une deuxième surface latérale (232) proche de la première poignée (13); et
une cavité de positionnement (25) formée dans la deuxième poignée (23) et comportant
une cavité d'insertion (252) renfoncée dans la première surface latérale (231) de la deuxième poignée (23) et comportant une surface d'extrémité éloignée de la première surface latérale (231) de la deuxième poignée (23) espacée de la deuxième surface latérale (232) de la deuxième poignée (23) et au moins une surface délimitant la cavité (253) définie dans une surface annulaire de la cavité d'insertion (252);
une tige filetée (30) comportant
une tête (31) montée dans la cavité d'insertion (252) de la cavité de positionnement (25) et comportant
au moins une surface délimitant la tête (312) définie dans une surface annulaire de la tête (31) et correspondant à l'au moins une surface délimitant la cavité (253) en termes de position et de forme; et
une tige (33) s'étendant de la tête (31) vers la première poignée (13) et s'étendant à travers la deuxième poignée (23) et la première poignée (13); et
un écrou (40) relié à la tige (33) de la tige filetée (30) via une liaison filetée et butant sélectivement contre la première poignée (13); et **caractérisé en ce que**
la cavité de positionnement (25) comprend plusieurs rainures d'engagement (255) disposées autour de la cavité d'insertion (252) et espacées les unes des autres, et chacune des rainures d'engagement (255) comportant une ouverture latérale formée dans la surface annulaire de la cavité d'insertion (252) et communiquant avec la cavité d'insertion (252) et une surface d'extrémité près de la première surface latérale (231) de la deuxième poignée (23) espacée de la première surface latérale (231) de la deuxième poignée (23); et
la tige filetée (30) comporte plusieurs saillies d'engagement (32) saillant à partir de la surface annulaire de la tête (31), espacées l'une de l'autre, et respectivement en prise avec les rainures d'engagement (255) de la cavité de positionnement (25).

2. Spéculum vaginal selon la revendication 1, dans lequel chacune des rainures d'engagement (255) possède une ouverture d'extrémité formée dans la deuxième surface latérale (232) de la deuxième poignée (23).

3. Spéculum vaginal selon la revendication 2, dans lequel chacune des saillies d'engagement (32) comporte une surface de guidage (322) définie dans une des deux extrémités de la saillie d'engagement (32) près de la tige (33).

4. Spéculum vaginal selon la revendication 3, dans lequel l'une des deux extrémités de chacune des saillies d'engagement (32) éloignée de la tige (33) est alignée avec l'une des deux extrémités de la tête (31) éloignée de la tige (33), et une distance est formée entre l'autre des deux extrémités de chacune des saillies d'engagement (32) près de la tige (33) et l'autre des deux extrémités de la tête (31) près de la tige (33).

5. Spéculum vaginal selon l'une quelconque des revendications 1 à 4, dans lequel
la cavité d'insertion (252) comporte deux dites surfaces délimitant la cavité (253) et deux surfaces incurvées de cavité (254) définies dans la surface annulaire de la cavité d'insertion (252), les deux surfaces délimitant la cavité (253) sont espacées l'une de l'autre, et les deux surfaces incurvées de cavité (254) sont reliées aux deux surfaces délimitant la cavité (253) et sont disposées à l'opposé l'une de l'autre;
la cavité de positionnement (25) comporte deux dites rainures d'engagement (255) respectivement adjacentes aux deux surfaces de cavité incurvées (254) et communiquant avec la cavité d'insertion (252);
la tête (31) de la tige filetée (30) comporte deux dites surfaces délimitant la tête (312) et deux surfaces de tête incurvées (313), les deux surfaces délimitant la tête (312) sont respectivement alignées avec les deux surfaces délimitant la cavité (253), et les deux surfaces incurvées de tête (313) sont reliées aux deux surfaces délimitant la tête (312), sont disposées à l'opposé l'une de l'autre et sont alignées respectivement avec les deux surfaces incurvées de cavité (254); et
la tige filetée (30) comporte deux dites saillies d'engagement (32) saillant respectivement à partir des deux surfaces incurvées de tête (313) et en prise respectivement avec les deux rainures d'engagement (255).

6. Spéculum vaginal selon la revendication 5, dans lequel
un trou de tige (24) est formé à travers la deuxième surface latérale (232) de la deuxième poignée (23) et la surface d'extrémité de la cavité d'insertion (252) et communique avec la cavité d'insertion (252);
un diamètre d'un cercle circonscrit du trou de tige (24) est inférieur à un diamètre d'un cercle circonscrit de la cavité d'insertion (252);
les rainures d'engagement (255) sont espacées du trou de tige (24);
un trou traversant (14) est formé à travers la première poignée (13); et
la tige (33) de la tige filetée (30) s'étend à travers le trou de tige (24) dans la deuxième poignée (23) et le trou traversant (14) dans la première poignée (13) et s'étend hors de la première poignée (13).
